# EUROPEAN PATENT APPLICATION

(11) **EP 1 013 299 A1**
(43) Date of publication of application: **28.06.2000**
(21) Application number: 97940335.9
(22) Date of filing: 10.09.1997
(51) Int. Cl.: A61M 5/315, A61M 5/24

(54) **SLIDE VALVE FOR SYRINGES, SYRINGE, AND KIT PREPARATIONS**

(30) Priority: 10.09.1996 JP 23941696
(71) Applicant: HIGASHIKAWA, Tetsuro, Meguro-ku, Tokyo 153 (JP)
(72) Inventor: HIGASHIKAWA, Tetsuro, Tokyo 153 (JP); SUZUKI, Hirokazu, Kanagawa 225 (JP)
(74) Representative: Weise, Reinhard, Dipl.-Ing.
(86) International application number: JP9703193
(87) International publication number: WO9810815

(57) **Abstract**

Use is made of a sliding valve for hypodermic syringe made of a rubber to which a lubricating component is chemically bonded, thereby acquiring a superior sliding valve for hypodermic syringe without using silicone coating together.

## Description

### [Technical Field]

The present invention relates to a sliding valve for use in hypodermic syringes such as a disposable syringe.

### [Background Art]

Injection agent introduced by injection into the body has hitherto been filled into ampuls or vials for supply. However, there arose problems that minute glass powders occurring at the time of unsealing the ampuls may possibly enter the interior of body or that various difficulties may possibly take place due to the contact of the medicines with the air. Hence, in recent years, it tends to be supplied in the form of so-called kit preparations in which medicines are previously filled into the disposable syringe.

Since the disposable syringe is discarded literally, it is currently widely used due to its convenience and to freedom from any fears of contagion of serum hepatitis. This one comprises a cylinder portion made of resin or glass and a plunger portion having a sliding valve made of rubber or plastic having rubber properties (hereinafter they are referred to as "rubber") .

Two different materials, i.e., glass and polypropylene were used to make the cylinder portion. Herein, the syringe having the glass made cylinder has been considered to be suitable for the kit preparations in particular due to its superior medicine resistance and gas barrier properties, although it involved problems of its high price and processing after use. On the contrary, the polypropylene made one is inferior in the anti-medicine properties and gas barrier properties but has an advantage of low price and easy post-use disposable by incineration.

The both have subjected the interior of their syringe bodies and the sliding valves to silicone coating in order to improve the sliding properties of the sliding valve.

Herein, the injection agent must be introduced into the interior of the body slowly in order to ensure safety and the effective action thereof, and hence the action of the sliding valve must be smooth. In the case of the kit preparations in which the liquid medicine is stored in the cylinder and in which the sliding valve is provided as the front stopper in addition to the provision within the plunger portion, the presence the two sliding valves within the interior of the cylinder caused remarkably lowered handing properties due to the deteriorated sliding properties, and hence the silicone coating for improving this played an extremely important role.

Recently, however, a question has been presented of the safety of the silicone coating. For this reason, an amorphous polyolefin (copolymer of cyclic olefin and α-olefin) made cylinder portion was proposed as solutions to the respective deficiencies of the glass made and polypropylene made cylinder portions with the unnecessary silicone coating. That is, dissimilar to the glass made syringe, the amorphous polyolefin enables its inner surface to be made into smooth mirror surface at the time of manufacture, whereby a good sliding property is assured with the unnecessary silicone coating.

Actual investigation has, however, proved that although a certain sliding property is obtained without the silicone coating, any sufficient sliding property required for the hypodermic syringe could not be obtained and that the silicone coating is still carried out at present.

On the contrary, as alternative to the silicone coating, measures have been taken in which the fluororesin layer is provided in the interior of the cylinder or on the surface of the sliding. In this case, however, an adhesion may often occur at the contact portion between the sliding valve and the interior of the cylinder, which was particularly problematic in cases where the sliding valve is held for a long period of time at the position other than the tip of the interior of the cylinder as in the kit preparations and where the injection must be made for the patient at that position. Furthermore, a minute crack may occur at the adhesion area between the cylinder interior and the sliding valve, presenting questions about the gas barrier properties as well as agent stability and safety.

Another method has been contrived in which a surface film is formed on the rubber made sliding valve, although it lacks in reliability due to difficult prevention of its peeling. On the other hand, there has also been proposed a sliding valve into which fine power of sliding material such as Teflon was mixed, although similarly there is a fear for the desorption of such fine powder.

In this manner, a safe sliding valve has been desired which ensures a full function of the sophisticated and complicated syringe having a good sliding property and which is free from any fear of peeling and desorption of foreign particles.

It is the object of the present invention to provide a hypodermic syringe which solves the above problems entailed in the above prior art and which has a sliding valve with a good sliding property and free from any peeling and desorption.

### [Disclosure of the Invention]

The sliding valve for hypodermic syringe of the present invention is a sliding valve for hypodermic syringe, made of a rubber to which a lubricating component is chemically bonded.

In the present invention, the rubber refers to a rubber or a plastic having a rubber property. This can be nitrile rubber, hydrogen added nitrile rubber, ethylene-propylene-diene rubber, ethylene-propylene rubber, etc. This one must be free from any fear of elusion of organic matter such as plasticizing agent or inorganic matter, and injurious metal, which may possibly adversely affect the living body, or it must be suitable for the medical applications without inclusion of such matters. Also, this one must have a medicine resistance against the objective medicine.

In the present invention, the lubricating component is preferably a synthetic oil based lubricating material. Such ones can include for example hydrocarbon based, polyglycol based or fluorine based ones. These lubricating components are induced and chemically bonded to the above rubber. The amount of lubricating component to be chemically bonded thereto must be determined through various investigations since it may influence the lubricating properties to a large extent.

The methods of chemical bonding may be achieved through common means such as ordinary amide bond, ester linkage, etc., although there can be employed a method in which a group reacting with a rubber cross linking agent is previously introduced into the lubricating component so that the chemical bonding is carried out simultaneously with the rubber crosslinking.

Also, in the case of a lubricating material with a less reactivity such as fluororesin (e.g., copolymer of polytetrafluoroethylene or tetrafluoroethylene with other comonomer), an electrophilic reagent (hindered phenolics, Grignard reagent) may be added together with these lubricating agents at the time of molding of the rubber, to achieve a chemical bonding simultaneously with the vulcanizing reaction.

The size of the lubricating material is preferably not less than 10 µm and not more than 50 µm in diameter. The loadings are preferably not less than 5 parts by weight and not more than 60 parts by weight, particularly preferably not less than 10 parts by weight and 50 parts by weight, with respect to 100 parts by weight of the rubber component.

The case of using the fluororesin as the lubricating component will hereinafter be described in detail. That is, use of the fluororesin as the lubricating component naturally ensures an easiness of injections such as intravenous injection at a predetermined point due to extremely smooth motion of the sliding valves as well as smooth starting motion thereof, simultaneously providing effects that a predetermined amount of injection is extremely easy to perform even in the case of kit preparations in which the amount of injection may be controlled in accordance with the patients instead of injecting all of the injection liquid residing within the cylinder.

Also, it could achieve easily the application of high viscous, high molecular weight sodium hyaluronate into the knee joint capsule, which was difficult to achieve by the conventional hypodermic syringe.

In the above, the electrophilic reagent must be compatible with the rubber component and must not injure the nature of the matrix rubber. Such ones can include hindered phenolics, for example, pentaerythrithyltetrakis [3 - (3, 5 - t - butyl - 4 - hydroxyphenyl) propionate], 2, 2 - thiodiethylenebis [3 - (3, 5 - di - t - butyl - 4 - hydroxyphenyl) propionate] 2, 2 - thiobis (4 - methyl - 6 - t - butylphenol) N, N' - bis [3 - (3, 5 - di - t - butyl - 4 - hydroxyphenyl) propionyl] hydrazine, and the Grignard reagent can be chlorine containing or iodine containing ones such as isopropyl magnesium chloride or isooctyl magnesium iodide.

Preferably, the composition of such electrophilic reagents is usually not less than 0.1 parts by weight and not more than 10 parts by weight, and particularly not less than 0.2 parts by weight and not more than 5 parts by weight, with respect to 100 parts by weight of the rubber.

These lubricating agents and electrophilic reagents are blended with rubber raw material (crude resin and vulcanizing agent) and are subjected to vulcanizing processing within, e.g., a metal mold at a temperature of the order of 150°C, thereby obtaining a sliding valve for hypodermic syringe in which the lubricating component is chemically bonded. It is to be noted that, in addition to the above, various modifiers could be added thereto if necessary although they must have safety as the materials for sliding valves for hypodermic syringe.

In the sliding valve for hypodermic syringe of the present invention, the lubricating component becomes integral with the rubber by virtue of the chemical bonding, with the result that there will be no fear that it may mix into the medicines through its eluation or peeling. Furthermore, the rubber itself has a lubricating property, so that there will be no need for silicone coating and for the provision of lubricating film or the like, which will render it safe.

The material of the cylinder portion forming a syringe in combination with the above sliding valve can be usually used ones, that is, glass, polypropylene or polyolefin based polymer such as amorphous polyolefin. Particularly desirable are ones having a cyclic olefin copolymer as unit since they satisfy the performances required for the hypodermic syringe such as safety, heat/cold resistance, transparency, high accuracy, gas barrier properties, medicine resistance, and others, with their easy thermal disposal after use.

### [Best Mode for Carrying Out the Invention]

### Examples (by ordinary hypodermic syringe; Examples 1 to 3 / comparative examples 1 to 4)

First, the operability of the sliding valve for hypodermic syringe of the present invention was evaluated with respect to the ordinary shaped hypodermic syringe. In this case, the rubber sliding valve to which the lubricating component in accordance with the present invention is chemically bonded was prepared as follows.

That is, an unvulcanized rubber compound was obtained by mixing, at a weight ratio shown in the composition 1 of Table 1, nitrile rubber (NBR, before vulcanization), stearic acid, zinc oxide, sulfur (colloidal sulfur), HAF carbon, di- (2-ethylhexyl) phthalate (DOP), N, N' - bis [3 - (3, 5 - di - t - butyl - 4 - hydroxyphenyl) propionyl] hydrazine, and as a vulcanization accelerator, N - cyclohexyl - 2 - benzothiazolil sulfenamide (CZ; manufactured by Kawaguchi Kagaku), dibenzothiazyl disulfide (DM; manufactured by Kawaguchi Kagaku), tetramethylthiuram disulfide (TT; manufactured by Kawaguchi Kagaku), and polytetrafluoroethylene powder (PTFE powder, particle diameter of not less than 20 µm and not more than 30 µm). The resultant compound was heated within the metal mold to a temperature of 180°C to form it as a sliding valve (example 1).

The other compounds having compositions shown in the compositions 2 to 5 of Table 1 were prepared and similarly vulcanized to obtain sliding valves (examples 2 and 3, and comparative examples 1 and 2).

These were subjected to evaluations as the sliding valves for hypodermic syringe, together with the first example sliding valve, the conventional silicone coated butyl rubber sliding valve (available from Maeda Sangyo; hereinafter referred to as "silicone coated butyl rubber sliding valve"; comparative example 3) and uncoated sliding valve (available from Maeda Sangyo; hereinafter referred to as "uncoated butyl rubber sliding valve"; comparative example 4).

**TABLE 1**

| | COMPOSITION 1 | COMPOSITION 2 | COMPOSITION 3 | COMPOSITION 4 | COMPOSITION 5 |
|---|---|---|---|---|---|
| nitrile rubber (NBR) | 100 | 100 | | 100 | 100 |
| ethylene propylene diene rubber (EPDM) | | | 100 | | |
| stearic acid | 1 | 1 | 1 | 1 | 1 |
| zinc oxide | 5 | 5 | 5 | 5 | 5 |
| sulfur | 2 | 2 | 2 | 2 | 2 |
| HAF carbon | 30 | 30 | 30 | 30 | 30 |
| DOP | 10 | 10 | 10 | 10 | 10 |
| N, N'-bis [3-(3, 5-di-t-butyl-4-hydroxyphenyl) propionyl] hydrazine | 0.5 | | | | |
| 2, 2-thiodiethylenebis[3-(3, 5-di-t-butyl-4-hydroxyphenyl) propionate] | | 0.5 | | | |
| isooctyl magnesium iodide | | | 0.5 | | |
| CZ | 1 | 1 | 1 | 1 | 1 |
| DM | 0.5 | 0.5 | 0.5 | 0.5 | 0.5 |
| TT | 0.2 | 0.2 | 0.2 | 0.2 | 0.2 |
| PTFE powder | 10 | 10 | 10 | 10 | |

As tests, monitor tests were carried out with ten physicians as monitors using a 5 ml of amorphous polyolefin made cylinder (available from Maeda Sangyo). Note that a silicone coated cylinder was used only in the case of the test using the silicone coated butyl rubber sliding valve whereas cylinders free from any silicone coating were used in the other cases.

A physiological salt solution was used as the injection liquid to perform a test with the assumption of intravenous injection. The feel of the plunger at the start of pushing (initial slidability), and the easiness (handling easiness) in the case of interruption at the amount of injection of 4ml were evaluated at 10 steps, with 10 being best, but 1 being worst, which results were averaged to round off the decimals. The results were shown in Table 2. Note that at that time no explanation on the details of samples was given to each monitor and that the tests were carried out in random order.

**TABLE 2**

| | Initial Slidability | Handling Easiness |
|---|---|---|
| sliding valve of composition 1 | 9 | 9 |
| sliding valve of composition 2 | 8 | 9 |
| sliding valve of composition 3 | 9 | 8 |
| sliding valve of composition 4 | 6 | 6 |
| sliding valve of composition 5 | 2 | 3 |
| silicone coated butyl rubber sliding valve | 6 | 6 |
| uncoated butyl rubber sliding valve | 2 | 2 |

Table 2 reveals that the operability in the sliding valves according to the compositions 1 to 3, which are sliding valves to which the lubricating component is bonded in accordance with the present invention, is remarkably satisfactory.

Although all of the sliding valves according to compositions 1 to 4 have powders of polytetrafluoroethylene with particle diameter of not less than 20 µm and not more than 30 µm, it is envisaged that desorption of these particles and introduction thereof into the body, in particular blood vessel may possibly impede the flow of blood. Herein, the investigation was carried out of the presence or absence of absorption from the sliding valve.

Three such sliding valves were put in the test tubes, to which 10 ml of physiological salt solution previously subjected to filtering by a filter with 0.45 µm pore diameter was added, which was then subjected to autoclaving for 20 minutes at 121°C. After having been cooled down to room temperature, the sliding valve was taken out, and thereafter the physiological salt solution was filtered by the filter with 0.45 µm pore diameter, to observe the filter surface by use of the scanning electron microscope. As a result, some particles (10 to 20 µm in diameter) were observed only in the case of using the sliding valve according to composition 4, and such particles were confirmed to allow an existence of fluorine by means of electron probe X-ray microanalyzer, although in the case of using the sliding valves according to the compositions 1 to 3 there was not found any particles on the filter. From this, it was confirmed that the sliding valves according to the compositions 1 to 3, which are sliding valves to which the lubricating component in accordance with the present invention is chemically bonded, completely prevents any desorption of the polytetrafluoroethylene powder.

### Example (by kit type hypodermic syringe; examples / comparative examples)

Examples of the kit type hypodermic syringe will be described.

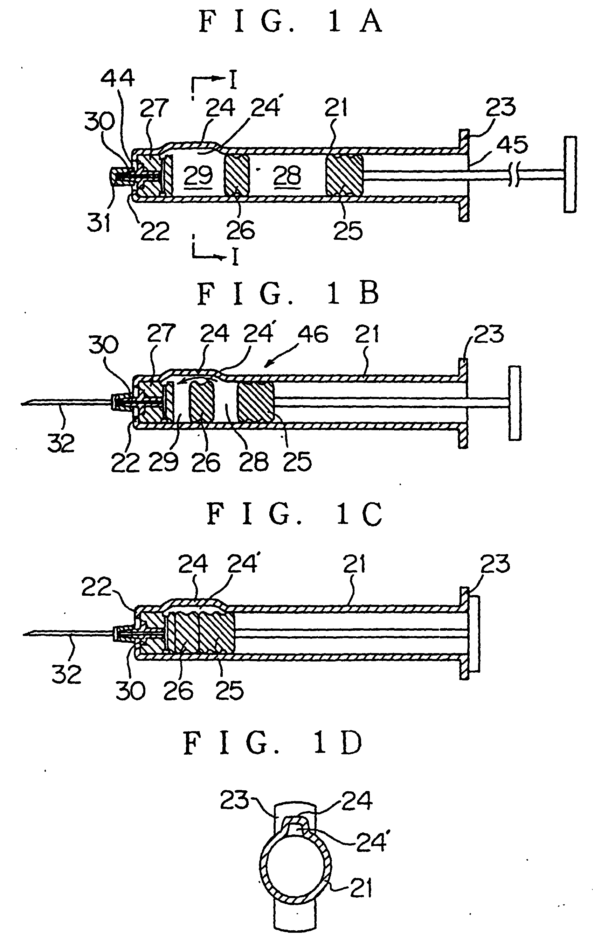

Figs. 1A to 1D show a kit type hypodermic syringe having a sliding valve for hypodermic syringe made of a rubber to which the lubricating component in accordance with the present invention is chemically bonded, respectively.

Fig. 1A shows the state of pre-use, Fig. 1B shows the state of in-use, Fig. 1C shows the state where the injection was complete, and Fig. 1D shows the sectional shape of the cylinder (sectional view taken along I-I in Fig. 1A).

In the diagram, refere, nce numeral 21 denotes a cylinder made of amorphous polyolefin and having openings 44 and 45 at its both ends, with the tip of the one opening 44 side being in the form of a hooked tip 22 which is hooked in section. Reference numeral 23 denotes a rear end portion of the cylinder, 24 denotes a bulged portion, and 25 denotes a plunger sliding valve. The bulged portion 24 as shown in Fig. 1D bulges at a part of the circumference of the cylinder 21, with the interior of the bulged portion being formed with a communication passage 24'.

In the vicinity of the center in the interior of the cylinder 21 there is fitted a front stopper sliding valve 26. The plunger sliding valve 25 and the front stopper sliding valve 26 are made of a rubber having the composition 1 in accordance with the present invention and have lips formed on their outer peripheral surfaces. The cylinder 21 at the left end of the diagram is water tightly sealed by an end partition 27 whereby within the cylinder there are formed a vacant chamber 28 defined between the plunger sliding valve 25 and the front stopper sliding valve 26 and a vacant chamber 29 defined between the end partition 27 and the front stopper sliding valve 26. An injection connecting portion needle 30 is fitted into the end partition 27 and is fitted with a cap 31.

The action of this hypodermic syringe will then be described. In Fig. 1A, safekeeping is effected in such a manner that an injection agent is filled into the vacant chamber 28 of the cylinder 21, with the vacant chamber 29 being filled with none. In use, the cap 31 is first removed, and the hypodermic needle 32 is fitted thereto in an lure lock method. Then, when the plunger is pressed as shown in Fig. 1B with the needle 32 directing upward, the front stopper sliding valve 26 is positioned in the communication passage 24', allowing the injection agent within the chamber 28 to move into the vacant chamber 29. The air within the vacant chamber 29 is fully exhausted and thereafter the hypodermic needle 32 is pierced through the skin of the patient. The injection agent flows through the interior of the hypodermic needle 32 and into the body of the patient.

Fig. 1C shows the state where the injection has been completed. The plunger is pushed in so that the end partition 27, the front stopper sliding valve 26 and the plunger sliding valve 25 are put in state of the mutual familiar contact. At that time, the tip of the plunger sliding valve 25 (closer to the hypodermic needle) reaches the passage 24', allowing all the injection agent within the chamber 28 to be fully injected. Furthermore, in order to ensure that the passage 27b of the end partition 27 also lies in the passage 24', determination is made of the axial thickness of the front stopper sliding valve 26 and the plunger sliding valve 25, as well as the length and the position of the bulged portion 24.

Such a hypodermic syringe moved the two sliding valves simultaneously upon the injection, which made it difficult to perform a minute action for intravenous injection or the like in the event of the hypodermic syringe free from application of the lubricating agent and requiring a certain force. However, the above example (example 1) in accordance with the present invention uses the sliding valve having a satisfactory lubricating property without any need for the lubricating agent, thereby ensuring an extremely smooth injection action as well as a slow and gradual introduction of the injection liquid into the body.

This kit type hypodermic syringe employed seven different sliding valves obtained through the same evaluations used for the ordinary hypodermic syringe, and using sodium hyaluronate having average molecular weight of 900,000 the monitor tests were carried out with the assumption of injection of the sodium hyaluronate into the knee joint capsules.

With the assumption of the kit preparations, a syringe having a 5ml of amorphous polyolefin cylinder into which 2.5ml of 1% sodium hyaluronate (having a viscous liquid state) was previously injected was experimentally fabricated as a kit type syringe shown in Figs. 1A to 1D using the above seven different sliding valves in accordance with a normal manufacturing method, the resultant syringe was left to stand for three months at a normal room temperature. These syringes were used to suck 1ml of lidocaine hydrochloride within the vial into each syringe, after which the evaluation was made with the assumption of full dose. Note that the silicone coated cylinder was used only in the case of the test using the silicone coated butyl rubber sliding valve, and that in the other cases use was made of cylinders subjected to no silicone coating.

The slidability of these sliding valves were evaluated in ten steps by ten monitors of orthopedic surgeons. 10 was best and 1 was worst, and the results were averaged to round off the decimals. The results are shown in Table 3.

**TABLE 3**

| | Operability at the time of Introduction of Lidocaine Hydrochloride | | Slidability upon Application |
|---|---|---|---|
| | Initial Slidability | Handling Easiness | |
| sliding valve of composition 1 | 9 | 8 | 6 |
| sliding valve of composition 2 | 10 | 9 | 6 |
| sliding valve of composition 3 | 9 | 9 | 6 |
| sliding valve of composition 4 | 6 | 6 | 3 |
| sliding valve of composition 5 | 2 | 3 | 1 |
| silicone coated butyl rubber sliding valve | 4 | 5 | 3 |
| uncoated butyl rubber sliding valve | 1 | 2 | 1 |

Table 3 reveals that in the case of using the sliding valves according to the compositions 1 to 3 which are the sliding valves in accordance with the present invention, the operability is excellent when safekept as kit preparations.

Particular attention is to be given to the fact that the superiority in the operability of the sliding vales according to the compositions 1 to 3 over the sliding valve according to the composition 4 similarly having the PTFE powder within the system arises from the chemical bonding fixation of the lubricating components of the sliding valves according to the compositions 1 to 3.

### [Example 4]

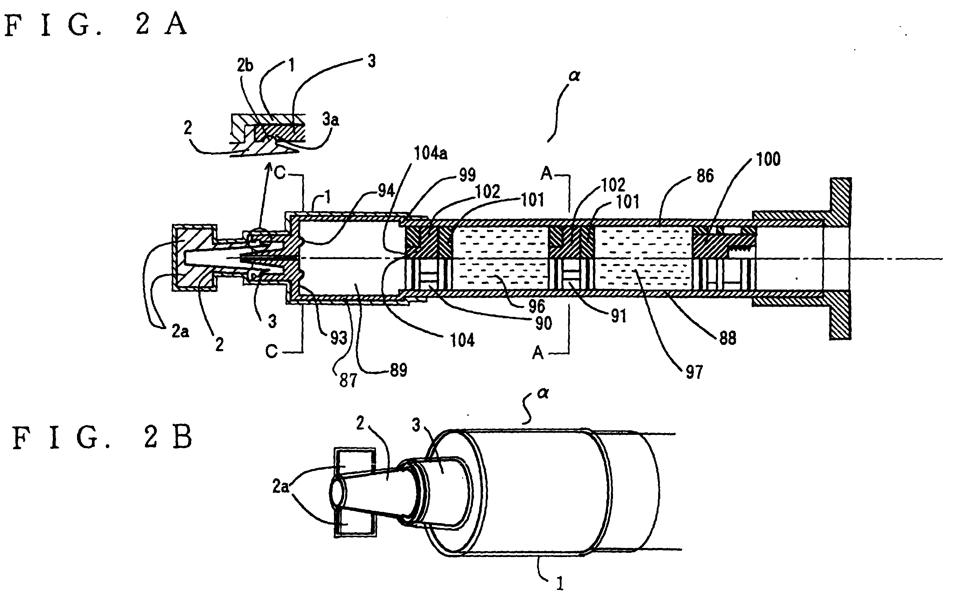

Fig. 2A shows as example 2 a kit type hypodermic syringe α having a sliding valve in accordance with the present invention and being subjected to seal peel processing.

In the diagram, designated at reference numeral 1 is a seal and the reference numeral 2 denotes a cap made of resin. A twist plate 2a is attached to the cap 2 to facilitate the removal of the cap 2 as will be described later.

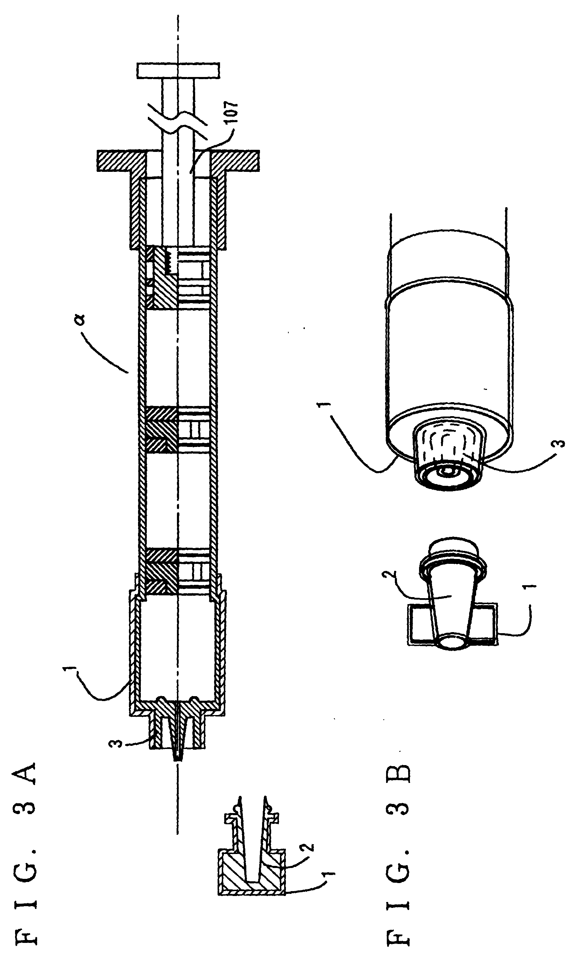

The tip of the kit hypodermic syringe α is of a lure lock type so that a lure lock portion 3 can prevent the hypodermic needle from being inadvertently disengaged. The lure lock portion 3 is formed with a groove 3a into which a raised portion 2b provided on the cap 2 fits. Note that the cap 2 is made of resin as described so that it can easily be removed from the lure lock portion 3 by twisting the twist plate 2a by fingers (see Fig. 2A and Fig. 2B. Note that Fig. 3A shows the state where a plunger rod 107 is mounted thereon).

The seal 1 is formed by dipping and, when the kit type hypodermic syringe α is sterilized by superheated steam, prevents the steam from entering the interior of the cap 2, the hypodermic needle connecting portion and the interior of the kit type hypodermic syringe α. The seal 1 further prevents germs from invading such spaces after sterilization. The material of the seal can be a photo-setting resin, a thermosetting resin, etc., and must be a material enduring the above sterilization conditions and ensuring the easy removal of the cap 2. The thickness of the seal 1 is selected in an appropriate manner, taking into consideration the above sterilization conditions and the easiness of removal of the cap 2. The seal 1 could be formed by a shrinkable film or the like satisfying the above conditions. In such a case, however, some film conditions might prevent a clean breakage at the boundary between the cap 2 and the lure lock portion 3, which will impede the connection between the needle and the syringe α, and hence appropriate measures must be taken.

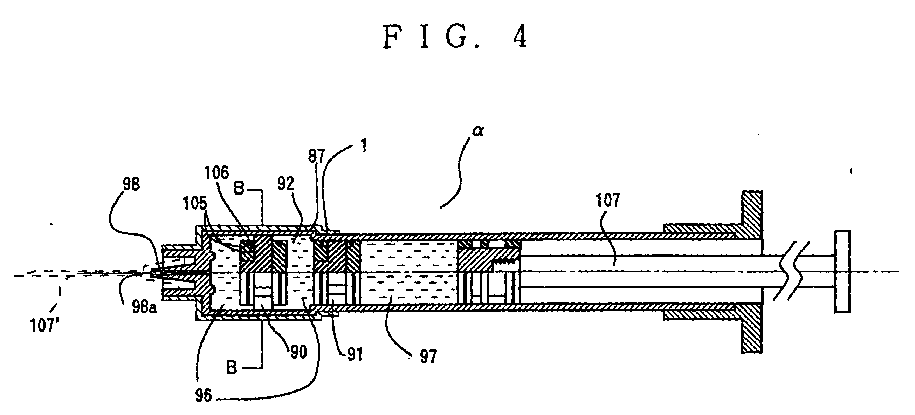

In Fig. 2A, arrangement is such that a larger diameter enlarged portion (bulged portion) 87 is integrally formed at the tip of the cylinder 86, with sliding valves 90 and 91 within the cylinder 86 being accommodated in a space 89 of circular section in the enlarged portion with a gap 92 remaining around the external diameter (Fig. 4, Fig. 9) and that the bottom wall 93 of the enlarged portion 87 is provided with a plurality of supporting protrusions 94 for the sliding valve 90 so that liquid medicines 96 and 97 within the cylinder 86 can be introduced from the gap 92 around the external diameter and a gap 95 (Fig. 9) defined by the protrusions 94 into a bore 98a of the needle connecting portion 98 (Fig. 4) following the enlarged portion 87.

The cylinder 86 may be made of glass, but it is most preferable to use synthetic resin material such as amorphous polyolefin having a good moldability and capable of presenting a mirror face configuration in order to obtain a good lubricating property.

In this example, the outer peripheral wall of the enlarged portion 87 is continuous integrally with the outer peripheral wall of a straight portion 88 by way of an annular shoulder 99.

Within the straight portion 88 of the cylinder 86 there are arranged two rubber made sliding valves (a front stopper 90 and a middle stopper 91) to constitute a series separate injection kit type hypodermic syringe α. In Fig. 2A, the front sliding valve 90 is positioned within the straight portion 88 slightly rearward of the enlarged portion 87, with the middle sliding valve 91 being at substantially the middle of the straight portion 88. A first liquid medicine 96 is filled into the space defined between the front sliding valve 90 and the middle sliding valve 91, while a second liquid medicine 97 is filled into the space defined between the middle sliding valve and a rear plunger (end stopper) 100. If for example the first liquid medicine 96 is a local anesthetic and the second liquid medicine 97 is sodium hyaluronate which is a therapeutic agent for osteoarthritis of knees, the pain of the patient would be reduced upon the administration of the therapeutic agent.

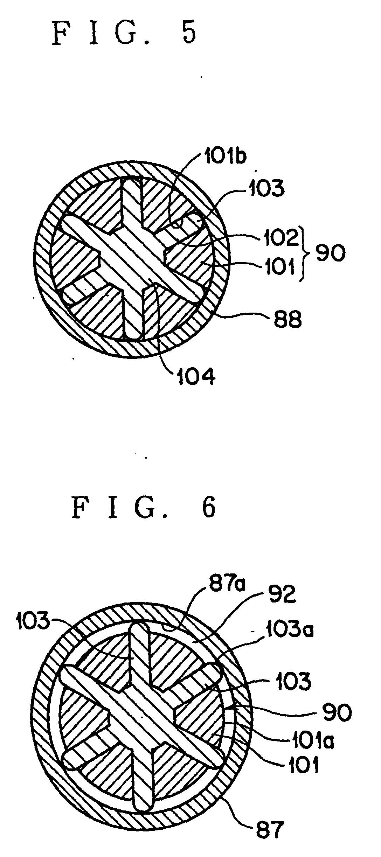

Both the sliding valves 90 and 91 as shown in Figs. 5 to 7 are made of a rubber (nitrile rubber) to which a lubricating component in accordance with the present invention is chemically bonded, and are constituted by two different rubber materials (a body 101 and a support member 102) depending on different crosslinking conditions. When the sliding valve 90 advances from the straight portion 88 of the cylinder 86 to the enlarged portion 87, a part 103 of the rubber member (support member) 102 on one hand which is soft and easy to expand or contract is radially projected to support the sliding valve 90 within the enlarged portion 87 of the cylinder 86 without any backlash, thereby keeping the gap 92 around the external diameter constant.

In Fig. 5, the rubber member (support member) 102 on one hand is incorporated in the straight portion 88 of the cylinder 86 with a higher compressibility, so that when the sliding valve 90 is put in a substantially released state as shown in Fig. 6, the rubber member (support member) 102 on one hand is allowed to elongate to a larger extent than the other rubber member (body) 101. Since the front sliding valve 90 and the middle sliding valve 91 are configured in the identical manner, the sectional shape and the function of the middle sliding valve 91 are also the same as Figs. 5 and 6.

Of the above sliding valves, the body portion 101 is obtained in the same manner as the sliding valve of the example 1.

It is to be noted that the body 101 is substantially in the form of a short cylinder and that the support member 102 consists of a substantially cylindrical central portion 104 positioned at the center of the body 101 and of a plurality of substantially fin-shaped expansion/ contraction portions (projecting portions) 103 which extend radially from the central portion 104 into the body 101.

As shown in Fig. 6, in their free state, the tips (supporting projections) 103a of the expansion/ contraction portions 103 project outwardly from the outer peripheral surface 101a of the body 101 and is in contact with the internal surface 87a of the cylinder enlarged portion 87. The supporting projections 103a have a semi-circular section and come into contact with the internal surface of the cylinder 86 with a lower sliding resistance.

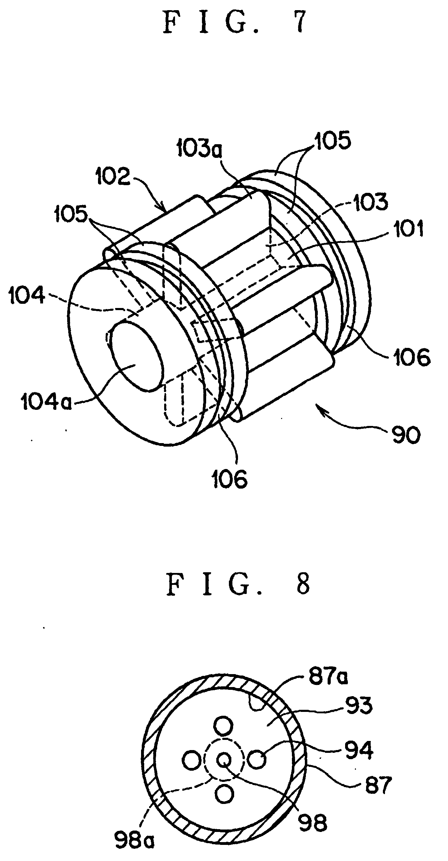

As shown in Fig. 7, the expansion/ contraction portions 103 of the supporting member 102 are disposed at the central portion in the axial direction of the body 101 in such a manner that the axial length of the expansion/ contraction portions 103 is greater than the plate thickness of the expansion/ contraction portions 103 to form substantially rectangular plates. The both ends in the axial direction of the body 101 are provided with an annular lip portion 105 making a sliding contact with the internal surface of the cylinder straight portion 88, and an annular groove 106 (Fig. 4) defined between the lip portions. The central portion 104 (Fig. 2) of the supporting member 101 extends in one axial direction to form a boss and allows its central portion tip surface 104a to be exposed at one end of the body 101. As shown in Fig. 7, the central portion of the body 101 is formed to have a diameter slightly smaller than the external diameter of the annular lip portion 105 at both ends.

The body 101 is formed from a rigid material presenting a less deformation and hard to expand or contract, whereas the supporting member 102 is formed from a flexible material presenting a larger deformation and easy to expand or contract, the supporting member 102 having an elasticity (a nature by which the object subjected to a change in shape or volume within a certain limit as a result of external application of force will restore its original state when the force is removed) identical to or larger than the body 101.

That is, the supporting member 102 is formed from a material having a lower modulus of elasticity (a higher compressibility) than the body 101. The body 101 and the supporting member 102 are in integrally intimate contact with each other.

It would also be possible to form the supporting member 102 from, e.g., a porous material having independent pores and a higher compressibility. The same material could also be used as long as they presented different compressibility (modulus of elasticity).

These sliding valves 90 and 91 may be formed by means of insert molding, which is well known in the injection molding technique, wherein the body 101 is first molded, and then the body 101 is inserted into another metal mold, into which the supporting member 102 is injection molded.

Although at the completion of the molding the body 101 and the support member 102 are brought into intimate contact with each other at the interfacial portion, part of the expansion/ contraction portions 103 is displaced along the side walls 101b of the body 101 to retract radially inward when the expansion/ contraction portions 103 of the supporting member 102 are pressed against the inner wall surface of the cylinder straight portion 88 as shown in Fig. 5. Then the outer peripheral surface (annular lip portion 105) of the body 101 comes into intimate contact with the internal surface 88a of the cylinder straight portion 88 so that the liquid medicine 95 is hermetically sealed as shown in Fig. 2. The front sliding valve 90 separates the liquid medicine 96 from the space 89 within the enlarged portion 87, to thereby prevent germs or the like from invading the liquid medicine 96.

This kit type hypodermic syringe α is provided with a plunger as shown in Figs. 3A to 3B so that the plunger is pressed to allow the sliding valve 90 to advance to the cylinder enlarged portion 87 as shown in Fig. 6, upon which the expansion/ contraction portions 103 project radially outward from the body 101 due to their high elasticity, thereby providing a stable support for the sliding valve 90 against the enlarged portion 87. Although they project outward of the body 101 due to their elasticity, the internal diameter of the cylinder enlarged portion 87 is set to be larger than the external diameter in the free state of the sliding valve body 101, with the result that between the adjacent expansion/ contraction portions 103 and 103 there are formed gaps 92 for the introduction of the liquid medicine between the cylinder enlarged portion 87 and the sliding valve body 101.

These gaps 92 will be sufficient if they are of the order of 0.1 to 0.5 mm. The tips (supporting projections) 103a of the radially extending expansion/ contraction portions 103 serve to keep the gaps 92 constant over the circumstance, thereby keeping the amount of flow and the rate of flow of the liquid medicine 96 so that the operating force of the plunger rod 107 (Fig. 4) is stabilized at a certain level. The number of the expansion/ contraction portions 103 may be at least three. If six is employed in this example, the posture of the sliding valve 90 within the enlarged portion 87 will become securely stabilized.

In Fig. 4, the first liquid medicine 96 flows through the gaps 92 around the external diameter of the front sliding valve 90 and is ejected from the bore 98a of the hypodermic needle connecting portion 98 into the hypodermic needle 107'.

Fig. 8 is a C-C sectional diagram of Figs. 2A to 2B, showing the supporting projections 94 formed integrally on the bottom wall 93 of the cylinder enlarged portion 87.

The projections 94 abut against the front end of the front sliding valve 90 to serve to provide the gaps 95 for the introduction of liquid medicine between the front sliding valve 90 and the enlarged portion bottom wall 93, the projections 94 being formed at substantially central position between the bore 98a of the hypodermic needle connecting portion 98 and the inner wall surface of the cylinder enlarged portion 87. This example employs four equally distributed projections 94 so as to able to provide a stable support for the front sliding valve 90. The number of the projections 94 is at least one and preferably three or more, with the height of the projections 94 being of the order of 1 mm.

Fig. 4 shows the state where from the state of Fig. 4, the plunger rod 107 is further pressed to complete the ejection of the liquid medicine 96, after which the middle sliding valve 91 is caused to advance into the enlarged portion 87 to start the ejection of the second liquid medicine 97.

As described hereinabove, the middle sliding valve 91 has the support member 102 within the interior of the body 101 in the same manner as the front sliding valve 90, so that the second liquid medicine 97 flows through the gaps 92 between the bodies 101 of the middle sliding valve 91 and of the front sliding valve 90 and the cylinder enlarged portion 87, and is ejected from the gaps 95 on the bottom wall 93 side of the enlarged portion 87.

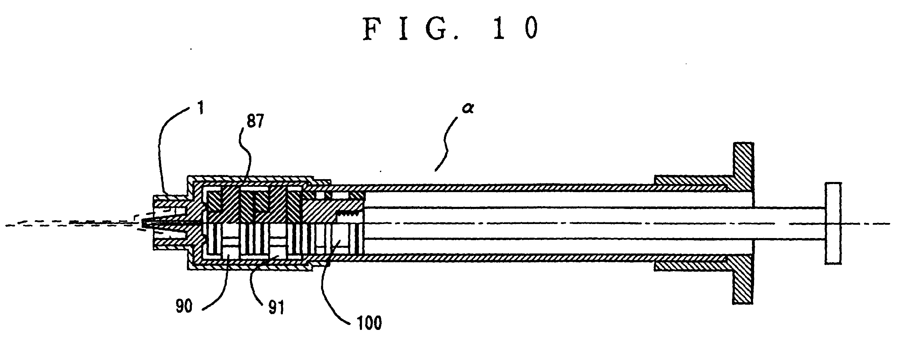

Fig. 10 shows the state where the ejection of the second liquid medicine 97 has been completed. The two sliding valves 90 and 91 are accommodated within the cylinder enlarged portions 87, and the plunger 100 abuts against the middle sliding valve 91 and stops.

Although the above embodiment shows the structure of a separate injection type hypodermic syringe using a plurality of sliding valves (made of a rubber to which a lubricating component is chemically bonded) 90 and 91, the syringe using the sliding valves made of a rubber to which a lubricating component in accordance with the present invention is chemically bonded is not limited to the separate injection type, it is also applicable to a normal one-liquid injection type kit syringe using a single sliding valve (front stopper 90) or to an in-use dissolution type kit syringe.

Although description has been made of the embodiments associated with the syringe whose cylinder (injection cylinder) is made mainly of amorphous polyolefin, the sliding valves of the present invention can conveniently be used for all types of syringes using cylinders made of other materials such as polyolefin including polypropylene or glass, in addition to such amorphous polyolefin cylinders.

### [Effect of the Invention]

By using the sliding valve for hypodermic syringe of the present invention, it is possible to eliminate the necessity for lubricating oil which may adversely affect the living body and the sliding materials or surface films which may peel off or separate, while ensuring a superior sliding property.

### [Brief Description of the Drawings]

Figs. 1A to 1D are a diagram showing a hypodermic syringe having a sliding valve of the present invention, respectively, in which Fig. 1A shows the state prior to the use, Fig. 1B shows the state where the injection is being carried out, Fig. 1C shows the state where the injection has been completed, and Fig. 1D shows the sectional shape of the cylinder.
Fig. 2A shows a kit type hypodermic syringe α having a sliding valve in accordance with the present invention and subjected to the seal peel processing. On the other hand, Fig. 2B shows the state of the vicinity of the tip of the kit type hypodermic syringe.
Fig. 3A is a diagram showing the state where a cap is removed from the kit type hypodermic syringe α. On the other hand, Fig. 3B shows the state of the vicinity of the tip of Fig. 3A.
Fig. 4 is a diagram showing the state where the hypodermic needle is fitted to the kit type hypodermic syringe α, with the air within the cylinder removed.
Fig. 5 is a sectional view showing the state where the sliding valve is positioned at a straight portion.
Fig. 6 is a sectional view showing the state where the sliding valve lies at the enlarged portion.
Fig. 7 is a perspective view showing the state where the sliding valve is positioned at the enlarged portion.

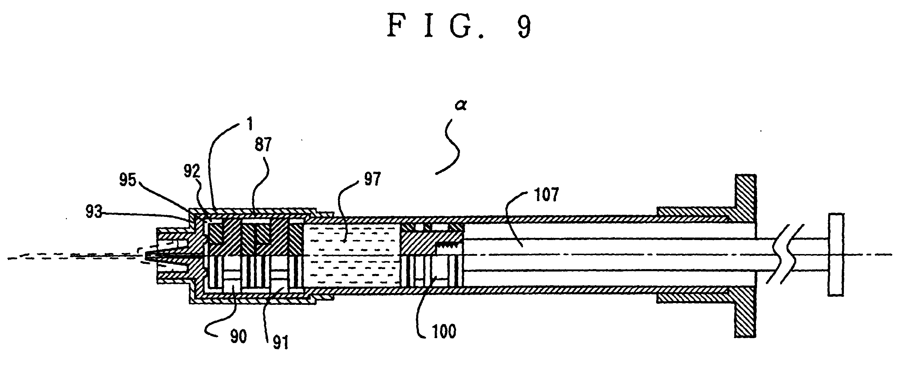

Fig. 8 is a diagram showing the supporting protrusion provided on the bottom wall.
Fig. 9 is a diagram showing the state where a part of the liquid medicine within the kit type hypodermic syringe has been injected.
Fig. 10 is a diagram showing the state where the injecting action has been completed.

### [Explanation of Reference Numerals]

21 cylinder
24 bulged portion
27 end partition
25 sliding valve of plunger
26 front stopper sliding valve
27b passage
28 chamber
30 hypodermic needle connecting portion
32 hypodermic needle
α kit type hypodermic syringe having a sliding valve in accordance with the present invention and subjected to seal peel processing
1 seal
2 cap
2a twist plate
2b raised portion
3 lure lock portion
3a groove
86 cylinder
87 enlarged portion
88 straight portion
90 front siding valve
91 intermediate sliding valve
93 bottom wall
94 supporting protrusion
96, 97 liquid medicine
98 hypodermic needle connecting portion
99 stepped portion
101 sliding valve body
101a outer peripheral surface
102 support member
103 expansion/ contraction portion
104 central portion
105 annular lip portion
106 annular groove
107 plunger rod

## Claims

1. A sliding valve for a hypodermic syringe made of a rubber to which a lubricating component is chemically bonded.

2. A sliding valve for a hypodermic syringe according to claim 1, wherein said lubricating component is polytetrafluoroethylene.

3. A sliding valve for a hypodermic syringe according to claim 1 or 2, wherein said lubricating component is chemically bonded by an electrophilic reagent.

4. A sliding valve for a hypodermic syringe according to any one of claims 1 to 3, wherein said electrophilic reagent is one or more selected from a group consisting of hindered phenolics and Grignard reagent.

5. A sliding valve for a hypodermic syringe according to claim 1, wherein it comprises two or more members having different moduli of elasticity.

6. A sliding valve for a hypodermic syringe according to claim 1, wherein it comprises two or more members having different compressibilities when stored in the cylinder portion.

7. A sliding valve for a hypodermic syringe comprising one or more selected from a group consisting of hindered phenolics and Grignard reagent, and a lubricating component.

8. A hypodermic syringe having a sliding valve made of a rubber to which a lubricating component is chemically bonded.

9. A hypodermic syringe having a cylinder made of a material selected from a group consisting of amorphous polyolefin, glass and polypropylene.

10. Kit preparations using a sliding valve made of a rubber to which a lubricating component is chemically bonded.

11. Kit preparations according to claim 10, further comprising a removable cap covering a hypodermic needle connection portion, and a seal for blocking the interior from the external air.

12. Kit preparations according to claim 11, further comprising a mechanism for facilitating the removal of said cap.
